(19) Europäisches Patentamt
European Patent Office
Office européen des brevets

(11) Publication number: **0 370 153 A1**

# (12) EUROPEAN PATENT APPLICATION

(21) Application number: 88402976.0

(22) Date of filing: 25.11.88

(51) Int. Cl.5: C07B 35/08, //C07C11/22, C07C15/48,C07C69/618, C07F7/18,C07D309/12, C07C1/26,C07C67/333

(43) Date of publication of application: 30.05.90 Bulletin 90/22

(84) Designated Contracting States: FR

(71) Applicant: MERRELL DOW PHARMACEUTICALS INC.
2110 East Galbraith Road
Cincinnati Ohio 45215-6300(US)

(72) Inventor: Van Hijfte, Luc
143, rue des Roses Wangen
F-67520 Marlenheim(FR)
Inventor: Kolb, Michael
16, rue du Kochersberg
F-67370 Truchtersheim(FR)

(74) Representative: Gillard, Marie-Louise et al
Cabinet Beau de Loménie 55, Rue d'Amsterdam
F-75008 Paris(FR)

## (54) Improvement process for debrominative rearrangements.

(57) This invention relates to an improvement in the standard procedures involving a debrominative rearrangement reaction of a terminal dibromoolefin with activated magnesium in tetrahydrofuran.

EP 0 370 153 A1

# IMPROVEMENT PROCESS FOR DEBROMINATIVE REARRANGEMENTS

This invention relates to improved chemical processes for the obtention of compounds having a terminally positioned acetylenic moiety.

More specifically, this invention relates to an improvement in the standard procedures involving a debrominative rearrangement reaction of a terminal dibromoolefin with activated magnesium.

As is well known in the art, preparation of compounds having a terminal acetylenic moiety have been prepared from dibromoolefins for quite some time. Indeed, the well known procedure described by Corey and Fuchs [see Tetrahedron Letters, 3769-72 (1972)], for the conversion of a dibromoolefin to its corresponding acetylenic derivative employs either n-butyllithium at -78 °C or lithium amalgam for the debrominative rearrangement step in yields which are generally considered good for this type of reaction. Unfortunately both reagents have disquieting drawbacks when large scale usage is envisioned. Problems with heat transfer, handling of large scale quantities of alkyl lithium with its inherently dangerous solvent-storage and general unavailability and high cost of lithium amalgam (a reagent prepared with some difficulties) are factors which are normally cited as drawbacks to the use of these reagents. Thus, we have been prompted to seek a more convenient reagent for this key reaction in the preparation of terminally positioned acetylene-containing compounds.

Another ramification of this improvement is its application for the conversion of aldehydes to terminal alkynes by a one carbon homologation. This particular application is suitable with compounds having a stereogenic center on the carbon atom adjacent the aldehyde. The stereogenic center is not epimerized during the two step transformation. It is especially suitable with compounds having alkyl-lithium sensitive groups.

Despite the negative results reported in the above Corey et al. reference, we have experimented with magnesium as a debrominative reagent and have developed a useful and practical improvement in this standard type of reaction.

In its generic aspects the invention involves the reaction

$$RCH{=}CBr_2 \xrightarrow[\text{reflux}]{\text{Mg, THF}} RC{\equiv}CH$$

and in its less generic aspect the invention involves the reaction sequence

$RCHO \rightarrow RCH{=}CBR_2 \rightarrow RC{\equiv}CH$

wherein R is a moiety of an organic compound having the depicted terminal aldehyde, dibromoolefin or acetylenic moieties attached thereto.

In effecting the process of this invention an activated magnesium is utilized in about equimolar quantities relative to the molar quantity of the dibromoolefin. In practice it is preferred to slowly add the dibromoolefin to the activated magnesium and the reactants are allowed to interact at about room temperature to about the reflux temperature of the reaction mixture, until the reaction is complete. In general it is preferred to add the olefin to the activated magnesium at such a rate as to encourage a gentle reflux although the reaction may be effected at about room temperature to about the reflux temperature. The reaction is normally completed in about 15 minutes to 4 hours, the time of course being dependent on the scale-up. Preferably minimum volumes of the anhydrous solvent is used but such quantity is not critical, only more convenient. Once the reaction is completed, as determined by standard techniques for monitoring such reactions, the reaction mixture is cooled, preferably to about room temperature and the magnesium bromide is removed by precipitation by the addition of solvent such as an alkane, preferably pentane, followed by filtration. Alternatively, the $MgBr_2$ can be removed by adding $H_2O$, followed by an extractive work up.

It is preferred to utilize freshly prepared activated magnesium. In practice, magnesium turnings and a trace of iodine in anhydrous tetrahydrofuran are heated at about reflux temperatures until the yellow color disappears. This iodine-activated magnesium is suitable for immediate use. Of course, other ways of activating the magnesium, such as trace amounts of an alkyl iodide, e.g. $CH_3I$ may be employed, or freshly prepared magnesium particles may also be used.

The dibromoolefins (II) may readily be prepared by standard procedures well known in the art. One preferred process for preparing the dibromoolefins is by subjecting the appropriate aldehyde (I) to a dibromomethylenation reaction using tetrabromomethane and triphenylphosphine according to standard procedures such as by contacting the reactants in a suitable solvent, e.g.$CH_2Cl_2$ at room temperature.

The following examples will serve to illustrate the techniques and processes described herein.

## EXAMPLE 1

**Activation of magnesium metal with iodine**

A mixture of magnesium turnings and a trace of iodine in anhydrous tetrahydrofuran are heated to reflux until the yellow color disappears. The obtained solution of iodine-activated magnesium metal in anhydrous tetrahydrofuran is used as such in each of the following examples.

**EXAMPLE 2**

**Preparation of 1-nonyne**

To 170 mg (7 mmol) of iodine-activated magnesium metal in 2 ml of anhydrous tetrahydrofuran is added a solution of 1.8 g (6.3 mmol) 1,1-dibromo-1-nonene in 6 ml of anhydrous tetrahydrofuran at such a rate to maintain a gentle reflux. The mixture is heated to reflux for 30 minutes and is then allowed to cool to room temperature. Pentane (8 ml) is added to precipitate the formed magnesium bromide, and the mixture is filtered through a short path of silica gel. The filter cake is rinsed with ether. The solvent is removed on the rotary evaporator, to obtain 720 mg (90%) of 1-nonyne.

**EXAMPLE 3**

**Preparation of 3-phenyl-1-butyne**

To 276 mg (11.4 mmol) of iodine-activated magnesium metal in 2 ml of anhydrous tetrahydrofuran is added 3 g (10.3 mmol) of 1,1-dibromo-3-phenyl-1-butene in 10 ml of anhydrous tetrahydrofuran, whereby a gentle reflux is maintained. The heating is continued for 30 minutes, the mixture is cooled down and 12 ml of pentane are added to precipitate the magnesium bromide. The solid is removed by filtration through a short path of silica gel, and the product is rinsed off with ether. After concentration *in vacuo* 1.1 g (80%) of pure 3-phenyl-1-butyne is obtained.

**EXAMPLE 4**

**Preparation of phenylacetylene**

A solution of 1.6 g (6.1 mmol) 2,2-dibromovinyl benzene in 5 ml anhydrous tetrahydrofuran is added dropwise to a mixture of 163 mg (6.7 mmol) iodine-activated magnesium metal in anhydrous tetrahydrofuran. The mixture is stirred for 30 minutes at room temperature, and 7 ml of pentane is added. The formed precipitate is removed by filtration through a short path of silica gel, and the reaction products are rinsed off with ether. After concentration *in vacuo* 530 mg (85%) of phenylacetylene, contaminated with 10% of styrene, is obtained.

**EXAMPLE 5**

**Preparation of 2-benzyl-4-pentyn-1-oic acid, t.butyl ester**

A solution of 1.0 g (2.5 mmol) of 2-benzyl-5,5-dibromo-4-pentene-1-oic acid, t.butyl ester in 3 ml of anhydrous tetrahydrofuran is added dropwise to a mixture of 70 mg (2.7 mmol) iodine-activated magnesium metal in 1 ml of anhydrous tetrahydrofuran. The mixture is stirred for 2 h at room temperature. 5 ml of pentane are added and the precipitated magnesium bromide is removed by filtration through a short path of silica gel. The reaction product is rinsed off with ether. The solvent is removed *in vacuo* to obtain 494 mg (82%) of pure 2-benzyl-4-pentyn-1-oic acid, t.butyl ester (the conversion of the 2-benzyl-5,5-dibromo-4-pentene-1-oic acid, t.butyl ester into 2-benzyl-4-pentyn-1-oic acid could not be accomplished using n.butyllithium in tetrahydrofuran).

**EXAMPLE 6**

Preparation of (3R,4R)3-t.Butyl dimethylsilyloxy-4-methoxy-4-methyl-1-octyne

Step A:

(2R,3S)2,3-Epoxy-3,7-dimethyl-6-octene-1-ol

A 2 liter 3-necked flask is fitted with an overhead mechanical stirrer, a thermometer and a pressure equalizing additional funnel. Activated crushed 3A molecular sieves (15 g) are brought in the flask, and the set up is dried under vacuum while heating with the heat gun. The flask is purged with nitrogen. Dry dichloromethane (dried over activated mol sieves, 600 ml) is introduced and the mixture is cooled to -15°C, utilizing a glycol/ water: 4/6 mixture-dry ice cooling bath or a cryocool apparatus. D-(-)-Diethyltartrate (77.8 mmol, 13.3 ml), titanium tetraisopropylate (58.3 mmol, 194 ml) and t.butylhydroperoxide (583 mmol, 194 ml of a 3 M solution in toluene (Fluka) are added sequentially). The mixture is agitated for 20 min at -15°C, and the temperature is lowered to -25°C. Nerol (389 mmol, 60 g, 68.5 ml) is added over a period of 30 min with vigorous stirring. The reaction mixture is kept for 3 h at -20°C. The cooling bath is removed, and the reaction mixture is allowed to warm to 0°C, at which moment water (340 ml) is added in one portion. After 15 min (internal temperature 17°C), a solution of 30% NaOH in concentrated NaCl (72 ml, prepared by adding 5 g NaCl to a solution of 30 g NaOH in 90 ml of water). A sudden phase separation occurs, and the stirring is stopped immediately. The mixture is transferred to an extraction funnel and the $CH_2Cl_2$ layer is removed. The water layer is extracted with $CH_2Cl_2$ (3 x 300 ml). Emulsions are broken by adding 15 ml of MeOH, while gentle stirring with a glass bar. The combined organic layers are washed with brine, dried over $MgSO_4$ and concentrated *in vacuo*. Distillation (95-97°C, 0.03 mmHg) affords 65.7 g (99%) of the desired epoxide $[\alpha]_D$ = +15.4°C (C = 3.3, $CHCl_3$).

Step B:

(2R,3S)2,3-Epoxy-3,7-dimethyl-6-octene-1-ol, benzyl ether

To a solution of (2R,3S)2,3-epoxy-3,7-dimethyl-6-octene-1-ol (386 mmol, 65.7 g) in dry tetrahydrofuran (400 ml), cooled in an ice-salt bath, is added benzyl bromide (463 mmol, 72.6 g), followed by the portionwise addition of potassium t.butoxide (463 mmol, 52 g), so that the temperature is kept below 10°C. The cooling bath is removed and the mixture is stirred overnight at room temperature. $NEt_3$ (15 l) is added and the mixture is stirred for an additional 24 h. Tetrahydrofuran is then evaporated and the residue is extracted with ethyl ether/water. The organic phase is dried over sodium sulfate and evaporated to afford (2R,3S)2,3-epoxy-3,7-dimethyl-6-octene-1-ol, benzyl ether as an oil. The crude ether is used without further purification in the next step.

Step C:

(2R,3R)1-Benzyloxy-3-methoxy-3,7-dimethyl-6-octene-2-ol

To a solution of the crude (2R,3S)2,3-epoxy-3,7-dimethyl-6-octene-1-ol, benzyl ether (120 g) in methyl alcohol (1 L) is added prewashed Dowex 50 (6 g). The mixture is stirred overnight at room temperature. TLC (petroleum ether/ethyl acetate: 80/20) indicates an uncomplete reaction. Dowex 50 is added, the reaction is stirred for an additional 24 h at room temperature. Dowex 50 is filtered and washed with dichloromethane. The organic solution, after addition of potassium carbonate (1 g), is kept without further purification for the next step.

Step D:

(2R,3R)2-Benzyloxymethyl-6-hydroxy-3-methoxy-3-methyl-tetrahydropyran

A 2 L 3-necked flask, fitted with a thermometer and a gas bubbler, is loaded with a solution of (2R,3R)1-benzyloxy-3-methoxy-3,7-dimethyl-6-octene-2-ol (0.098 mol) in 750 ml of a $MeOH/CH_2Cl_2$: 1/1 mixture. The acid-free reaction mixture is cooled in a dry ice-acetone bath (internal temperature approximately -60°C), and ozone bubbled through the solution. The mixture is stirred with a magnetic stirrer. After approximately 1 $\frac{1}{2}$ h, the solution turns blue, and the ozone bubbling is stopped.

A few drops of dimethylsulfide are added so that the blue color disappears (reduction of excess ozone). Triphenylphosphine (0.1 mol, 26.3 g) is added in one portion, and the cooling bath is removed. The mixture is allowed to warm slowly to room temperature and the stirring is continued for 1 h. The solvent is removed on the rotary evaporator. Finally, traces of methanol are removed by addition and evaporation of toluene (2 times with 100 ml). The residue is taken up in 100 ml of anhydrous tetrahydrofuran, and the product (2R,3R)2-benzyloxymethyl-6-hydroxy-3-methoxy-3-methyl-tetrahydropyran is used as such in the next reaction.

Step E:

(2R,3R)1-Benzyloxy-3-methoxy-3-methyl-6-heptene-2-ol

A dry 4 liter 3-necked flash, fitted with a thermometer, mechanical stirrer and addition funnel, is loaded with $Ph_3PCH_3Br$ (1.5 eq., 0.588 mol, 210 g),

dried overnight at 80° C under vacuum. The system is purged with nitrogen. Anhydrous tetrahydrofuran (600 ml) is introduced, the reaction mixture is cooled in a crushed ice-water bath, and KOt.Bu (2 eq., 0.784 mol, 88 g) is added (the mixing is exothermic) to give a bright yellow solution. The cooling bath is removed, and (2R,3R)2-benzyloxymethyl-6-hydroxy-3-methoxy-3-methyl-tetrahydropyran is added dropwise over $\frac{1}{2}$ h via an addition funnel with tetrahydrofuran (150 ml). The mixture is stirred for 3 h at room temperature. The reaction is cooled in an ice-water bath, and 800 ml of a saturated $NH_4Cl$ solution is added. The mixture is extracted with ethyl acetate, the water layer is removed and extracted once with ethyl acetate. The organic phase is washed with water and brine. After drying over sodium sulfate, the solvent is removed on the rotary evaporator. The residue is taken in petroleum ether (scratching and mixing precipitate the $Ph_3P=0$). Filtration of the triphenylphosphine oxide, washing 3 times with petroleum ether, followed by evaporation of the organic phase, affords the desired alkene as an oil. The alkene (2R,3R)1-benzyloxy-3-methoxy-3-methyl-6-heptene-2-ol is purified by filtration through a short path of silica gel, eluting successively with 95/5 petroleum ether/ethyl acetate (to rinse off the $Ph_3P$) followed by elution with petroleum ether/ethyl acetate: 70/30 yields the reaction product (2R,3R)1-benzyloxy-3-methoxy-3-methyl-6-heptene-2-ol as a yellow oil.

Step F:

(2R,3R)1-Benzyloxy-2-t.butyl dimethylsilyloxy-3-methoxy-3-methyl-6-heptene

To a solution of (2R,3R)1-benzyloxy-3-methoxy-3-methyl-6-heptene-2-ol (51.37 g, 194.3 mmol) in anhydrous difluoromethane (500 ml) at room temperature under nitrogen, is added imidazole (2 eq, 26.44 g, 388.5 mmol) followed by t.butyl dimethylsilyl chloride (1.5 eq, 44 g, 291.5 mmol). The mixture is stirred for 2 days. Dichloromethane is removed under reduced pressure, water (1 L) is added, and the mixture is extracted with ethyl ether (2 x 500 ml). The combined organic fraction is washed with 1 N HCl, then saturated $NaHCO_3$ and brine. After drying over sodium sulfate, the solution is evaporated. The residue is filtered through a short path of silica gel, first eluting with petroleum ether to remove the siloxanes, then with petroleum ether/ethyl acetate: 90/10 to collect the silyl ether (2R,3R)1-benzyloxy-2-t.butyl dimethylsilyloxy-3-methoxy-3-methyl-6-heptene (60.6 g, 82.3 %).

Step G:

(2R,3R)2-t.Butyl dimethylsilyloxy-3-methoxy-3-methyl-1-heptanol

A solution of (2R,3R)1-benzyloxy-2-t.butyl dimethylsilyloxy-3-methoxy-3-methyl-6-heptene (60 g, 158.6 mmol) and Pd(OH)2 on carbon (6 g) in ethyl acetate (600 ml) is hydrogenated at 1 atmosphere overnight. The reaction can be monitored by TLC. The catalyst is removed by filtration through a short path of celite. The filtrate is evaporated and the alcohol (2R, 3R)2-t.butyl dimethylsilyloxy-3-methoxy-3-methyl-1-heptanol is filtered through a short path of silica gel, eluting with petroleum ether/ethyl acetate: 80/20 to afford the desired product (2R,3R)2-t.butyl dimethylsilyloxy-3-methoxy-3-methyl-1-heptanol of this step as an oil.

Step H:

(2R,3R)2-t.Butyl dimethylsilyloxy-3-methoxy-3-methyl-heptanealdehyde

A solution of (2R,3R)2-t.butyl dimethylsilyloxy-3-methoxy-3-methyl-1-heptanol (43 g, 148 mmol) in dry dichloromethane (750 ml) under nitrogen is cooled in a dry ice-acetone bath. Oxalyl chloride (1.3 eq, 16.12 ml) is added dropwise. During the addition, gas evolution occurs. The mixture is stirred for 15 min, and triethylamine (4.5 eq, 88 ml) is added in one portion (a white solid precipitates). The cooling bath is removed to allow the mixture to warm to room temperature. Ethyl ether (2 L) is added, and water (4 L) to dissolve the precipitate. The organic phase is washed with 1 N HCl, saturated $NaHCO_3$ and brine. After drying over sodium sulfate, the solvent is removed on the rotary evaporator. The residue is taken up in a small amount of toluene and is concentrated again. The product (2R,3R)2-t.butyl dimethylsilyloxy-3-methoxy-3-methyl-1-heptanaldehyde is used as such in the next reaction.

Step I:

(3R,4R)1,1-Dibromo-3-t.butyl dimethylsilyloxy-4-methoxy-4-methyl-1-octene

To a solution of tetrabromomethane (2 eq, 98.28 g, 296 mmol) in dry dichloromethane (250 ml), under nitrogen, at 0° C is added dropwise a

solution of $Ph_3P$ (4 eq, 155 g, 592 mmol) in dry dichloromethane (250 ml). The speed of addition is regulated so that the temperature of the reaction stays below 20° C. The solution is orange-red. After 10 min, the cooling bath is removed and the aldehyde (2R,3R)2-t.butyl dimethylsilyloxy-3-methoxy-3-methylheptanaldehyde in solution in dichloromethane (100 ml) is added dropwise. The mixture is stirred at room temperature for 3 h. The reaction is cooled at -20° C, and triethylamine (4 eq, 88ml, 636 mmol) is added, followed by the very slow addition of water (300 ml), that gives an exothermic reaction. Precipitation occurs, water (2 L) is added to dissolve the precipitate, then the compound is extracted with dichloromethane (3 L). The organic phase is washed with 1 N HCl, saturated $NaHCO_3$, and brine. After drying over sodium sulfate, the solvent is evaporated. The residue is triturated with petroleum ether or precipitate triphenylphosphine oxide which is filtered off. The solvent is removed under reduced pressure. A second treatment with petroleum ether precipitates a second crop of triphenylphosphine oxide which is filtered again. Finally, the solvent is evaporated and the residue is purified by filtration through a short path of silica gel, eluting with petroleum ether/ethyl acetate (90/10) to yield the desired vinyl dibromide (3R,4R)1,1-dibromo-3-t.butyl dimethylsilyloxy-4-methoxy-4-methyl-1-octene (60 g, 91%).

Step J:

<u>(3R,4R)3-t.butyl dimethylsilyloxy-4-methoxy-4-methyl-1-octyne</u>

Magnesium turnings (1.3 eq, 4.2 g, 175 mmol) in anhydrous tetrahydrofuran (50 ml) are activated with a trace of $I_2$ or $CH_3I$. A solution of the vinyl dibromide (3R,4R)1,1-dibromo-3-t.butyl dimethylsilyloxy-4-methoxy-4-methyl-1-octen (60 g) in anhydrous tetrahydrofuran (600 ml) is added at such a rate to maintain a gentle reflux (some heating may be necessary). Stirring is continued at room temperature for 2 h. Ethyl ether (2 L) is added, the organic layer is washed with 1 N HCl, saturated $NaHCO_3$ and brine. The solution is dried over sodium sulfate and evaporated. The oil is distilled using a small Vigreux column at 66° C under 0.01 mmHg to yield the desired product (3R,4R)3-t.butyl dimethylsilyloxy-4-methoxy-4-methyl-1-octyne (31.4 g, 81.7%). GC:: 94% purity.

## EXAMPLE 7

**<u>Preparation of (3R,4R)3-Tetrahydropyranyloxy-4-methoxy-4-methyl-1-octyne</u>**

Step A:

<u>(2R,3R)1-Benzyloxy-3-tetrahydropyranyloxy-3-methoxy-3-methyl-6-heptene</u>

A solution of (2R,3R)1-benzyloxy-3-methoxy-3-methyl-6-heptene-2-ol (37.1 mmol, 9.8 g), dihydropyran (55.7 mmol, 5.1 ml) and pyridinium paratoluene sulfonate (3.71 mmol, 0.93 g) in anhydrous dichloromethane (100 ml) is stirred under nitrogen atmosphere for 24 h. Ether (400 ml) is added, and the organic layer is washed with water, brine, saturated aqueous $NaHCO_3$ and brine. After drying over anhydrous magnesium sulfate and solvent is flash evaporated. The product (2R,3R)1-benzyloxy-3-tetrahydropyranyloxy- 3-methoxy-3-methyl-6-heptene is used as such in the next reaction.

Step B:

<u>(2R,3R)2-Tetrahydropyranyloxy-3-methoxy-3-methyl-1-heptanol</u>

A mixture of (2R,3R)1-benzyloxy-2-tetrahydropyranyloxy-3-methoxy-3-methyl-6-heptene (1.78 mmmol, 620 mg) and $Pd(OH)_2$ on carbon (60 mg) in methanol (8 ml) is stirred 3 h under a hydrogen atmosphere whereby 82 ml of hydrogen is absorbed. The catalyst is filtered, washed with methanol and the methanol is removed *in vacuo*. The alcohol (2R,3R)2-tetrahydropyranyloxy-3-methoxy-3-methyl-1-heptanol is used as such in the next reaction.

Step C:

<u>(2R,3R)2-Tetrahydropyranyloxy-3-methoxy-3-methyl-heptanaldehyde</u>

To a solution of (2R,3R)2-tetrahydropyranyloxy-3-methoxy-3-methyl-1-heptanol (0.89 mmol, 230 mg) and dimethylsulfoxide (1.78 mmol, 0.13 ml) in anhydrous dichloromethane (5 ml) at -78° C under $N_2$ atmosphere is added dropwise a solution of oxalylchloride (1.16 mmol, 0.1 ml) in dichloromethane (2 ml). The solution is stirred 15 min, and triethylamine (3.56 mmol, 0.5 ml) is added.

The mixture is kept 10 min at -78°C, and is then allowed to warm to room temperature. Water (10 ml) and ether (50 ml) are added. The organic layer is separated and washed with 1 N HCl, saturated aqueous $NaHCO_3$ and brine. Drying over anhydrous magnesium sulfate and concentration gives a colorless oil, which is used as such in the next reaction.

Step D:

(3R,4R)1,1-Dibromo-3-tetrahydropyranyloxy-4-methoxy-4-methyl-1-octene

To a solution of CBr4 (1.78 mmol, 0.59 g) in dichloromethane (3 ml) at 0°C is added triphenylphosphine (3.56 mmol, 0.93 g) in dichloromethane (3 ml). The orange-red solution is stirred for 30 min and a solution of (2R,3R)2-tetrahydropyranyloxy-3-methoxy-3-methyl-heptanaldehyde (0.89 mmol, crude from previous reaction) in dichloromethane is added. After 1 h triethylamine (3.56 mmol, 0.5 ml) is added, followed by the slow addition of water (10 ml) (exothermic). Ether (50 ml) is added, the water layer is removed and the organic layer is washed with brine, dried over anhydrous $MgSO_4$ and concentrated *in vacuo*. Hexane is added to precipitate the triphenylphosphine oxide, and the mixture is filtered through a short path of silica gel; the filter cake is washed with 30% ether in hexane. The oil obtained after flash evaporation (240 mg, 65%) is used without purification in the reactions to prepare (3R,4R)3-tetrahydropyranyloxy-4-methoxy-4-methyl-1-octyne.

Step E:

(3R,4R)3-Tetrahydropyranyloxy-4-methoxy-4-methyl-1-octyne

A solution of (3R,4R)1,1-dibromo-3-tetrahydropyranyloxy-4-methoxy-4-methyl-1-octene (240 mg; 0.58 mmol) in tetrahydrofuran (2 ml) is added dropwise to a refluxing mixture of activated magnesium turnings (activated with $I_2$, 183 mg, 0.75 mmol) in tetrahydrofuran (1 ml). The mixture is refluxed for 1 h, hexane (5 ml) is added and the suspension is filtered through a short path of silica gel. The filter cake is washed with ether and the solvent is removed *in vacuo*. The alkyne (3R,4R)3-tetrahydropyranyloxy-4-methoxy-4-methyl-1-octyne is purified by column chromatography on silica gel, eluting with 5% EtOAc in petroleum ether, to afford (3R,4R)3-tetrahydropyranyloxy-4-methoxy-4-methyl-1-octyne.

In addition to the application of the instant improvement process for the preparation of the foregoing specific examples, the process is also applicable to the preparation of the following known pharmaceutical compounds from the corresponding dibromo olefin:

1. 8-[4-(4-Fluorophenyl)-1-methylbutyl]-1,3,4,5-tetrahydro-5,5-dimethyl-2-(2-propynyl)-2H-[1]benzopyrano-[4,3-c]pyridin-10-ol,
2. N-Methyl-N-(2-propynyl)-benzeneethanamine,
3. 5-[[(1,1-Dimethyl-2-propynyl)amino]-sulfonyl]-N-[(1-ethyl-2-pyrrolidinyl)methyl]-2-methoxybenzamide,
4. α-[[(1-Ethynylcyclohexyl)oxy]methyl]-4-[3-(trifluoromethyl)phenyl]-1-piperazineethanol,
5. 2,3-Dihydro-N-methyl-N-(2-propynyl)-1H-indene-1-amine,
6. α-Methyl-N-methyl-N-(2-propynyl)-benzeneethanamine,
7. 4-Azido-6-phenyl-5-(2-propynyl)-2-pyrimidinamine,
8. 4,4a,4b,5,6,7,8,8a,9,10-Decahydro-7-(1-hydroxy-1-methyl-2-propynyl)-2(3H)-phenanthrenone,
9. 4-Amino-5-hexynoic acid, (inner salt),
10. 2-Propynoic acid,
11. 1-(2-Phenylethyl)-4-(2-propynyl)-4-piperidinol propanoate,
12. Propargylglycine,
13. 7-Chloro-4,5-dihydro-5-phenyl-1-(2-propynyl)-3H-[1,4]benzodiazepine-2(1H)-one,
14. N-Methyl-N-(2-propynyl)-benzenemethanamine,
15. 1-[(1,1-Dimethylethyl)amino]-3-[2-(2-propynyloxy)phenoxy]-2-propanol,
16. α-Phenyl-α-(2-propynyloxy)-benzeneacetic acid, 2-(dimethylamino)ethyl ester,
17. Cis-1-[[2-(2,4-dichlorophenyl)-4-[(2-propynyloxy)methyl]-1,3-dioxolan-2-yl]methyl]-1H-imidazole,
18. Paraconazole,
19. 5-(Hepta-1,2-dien-4,6-diynyl)tetrahydro-2-furanone,
20. 1-Piperidinebutanoic acid, 8-(1,2-dimethylheptyl)-1,3,4,5,-tetrahydro-5,5-dimethyl-2-(2-propynyl)-2H-[1]benzopyrano[4,3-c]pyridin-10-yl ester,
21. 1,2,3,4-Tetrahydro-N-methyl-N-(2-propynyl)-naphthalenamine,
22. 3-Methyl-1-pentyl-3-ol,
23. 3-Methyl-1-pentyl-3-ol, carbamate (ester),
24. 6-Heptyn-2,5-diamine,
25. N-[3-Cyano-4-[3-(1,1-dimethyl-2-propynyl)amino]-2-hydroxypropoxy)phenyl]-2-methylpropanamide,

26. 2-Hydroxy-N-(1-methylethyl)-3-[2-(2-propynyloxy)phenoxy]propanaminium,

27. N-Methyl-N-(2-propynyl)-1H-inden-1-amine,

28. 2-(But-1-en-3-ynyl)-6′-(pent-2-en-4-ynyl)-spiro[cyclonexane-1,2′-piperidine],

29. 1-(2-propynyl)cyclohexyl carbamate,

30. Hexahydro-2,6-methano-11-methyl-6-phenyl-3-(2-propynyl)-3-benzazolin-8-ol, methanesulfonate (salt),

31. 4-Methyl-7-(2-propynyloxy)-2H-1-benzopyran-2-one,

32. Dodecahydro-6-(2-penten-4-ynyl)pyrrolo-[1,2-a]quinoline-1-ethanol,

33. 1,2-Benzenedicarboxylic acid, 1-ethyl-1-methyl-2-propynyl ester,

34. 1-Ethynyl-2′-fluorobiphenyl,

35. α-[(1-Ethynyl-1-cyclohexyloxy)methyl]-4-(4-fluorophenyl)piperazineethanol,

36. N′-Cyano-N-[2-[[(5-methyl-1H-imidazol-4-yl)methyl]thio]ethyl-N″-(2-propynyl)guanidine,

37. 1-Ethynylcyclohexyl carbamate,

38. 1-Chloro-3-ethylpent-1-en-4-yn-3-ol,

39. 3-Methoxy-p-aza-19-norpregna-1,3,5(10)-trien-20-yn-17-ol,

40. Cyclohexylcarbamic acid 1,1-diphenyl-2-propynyl ester,

41. α,N-Dimethyl-N-(2-propynyl)-benzeneethanamine,

42. N-[3-[(2,4-Dichlorophenoxy)propyl]]-N-methyl-2-propyn-1-amine,

43. β-Hydroxy-α-methyl-α-ethynylbenzeneethanol,

44. N-[4-[[2-Amino-1,4-dihydro-4-oxo-6-quinazolinyl)methyl]-2-propynylamino]benzoyl]-L-glutamic acid,

45. α-Ethynylbenzenemethanol carbamate,

46. N-(1,1-Dimethylethyl)-N,2-dimethyl-3-butyn-2-amine,

47. N-(4-Chlorophenyl)-N′-methyl-N′-(1-methyl-2-propynyl)urea,

48. 1,2,5,6-Tetrahydro-1-methyl-3-pyridinecarboxylic acid, 2-propynyl ester,

49. 2,3-Dihydro-N-(2-propynyl)-1H-inden-1-amine.

Although the foregoing examples illustrate the application of the instant process, it is not contemplated that the improved process is only suitable for use in the pharmaceutical art. Indeed, this process is applicable to all organic compounds to which the above-mentioned debrominative rearrangement process of Corey and Fuchs may be theoretically useful.

## Claims

1. In the process for effecting a debrominative rearrangement reaction according to the reaction scheme

$RCH=CBR_2 \rightarrow RC\equiv CH$,

wherein R is a moiety of an organic compound bearing the depicted dibromoolefin improvement which comprises conducting the reaction in the presence of activated magnesium in anhydrous tetrahydrofuran at temperatures in the range of about 0°C to about the reflux temperature of the reaction mixture.

2. A process according to Claim 1 wherein an iodine-activated magnesium is utilized.

3. A process according to Claim 1 wherein a methyl iodide-activated magnesium is utilized.

European Patent Office

# EUROPEAN SEARCH REPORT

Application Number

EP 88 40 2976

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | BULLETIN OF THE ACADEMY OF SCIENCES OF THE USSR, DIVISION OF CHEMICAL SCIENCE, vol. 32, no. 10, part 2, October 1983, pages 2160-2162, Plenum Publishing Corp.; L.I. ZAKHARKIN et al.: "Formation of 1-alkynes by action of magnesium on the 1,1-dichloro- and 1,1-dibromo-1-alkenes in tetrahydrofuran solution" * Whole article * --- | 1-3 | C 07 B 35/08 //<br>C 07 C 11/22<br>C 07 C 15/48<br>C 07 C 69/618<br>C 07 F 7/18<br>C 07 D 309/12<br>C 07 C 1/26<br>C 07 C 67/333 |
| Y | SYNTHESIS, no. 8, August 1981, pages 585-604, Georg Thieme Verlag; Y.-H. LAI: "Grignard reagents from chemically activated magnesium" * Pages 585,586 * --- | 1-3 | |
| D,A | TETRAHEDRON LETTERS, no. 36, September 1972, pages 3769-3772, Pergamon Press; E.J. COREY et al.: "A synthetic method for formyl-ethynyl conversion (RCHO-RC=CH or RC=CR')" * Whole article * ----- | 1-3 | TECHNICAL FIELDS SEARCHED (Int. Cl.5)<br>C 07 B 35/00<br>C 07 C 1/00<br>C 07 C 11/00<br>C 07 C 15/00 |

The present search report has been drawn up for all claims

| Place of search | Date of completion of the search | Examiner |
|---|---|---|
| THE HAGUE | 10-07-1989 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document